# FASCICULE DE BREVET EUROPEEN

(11) **EP 0 682 873 B1**
(45) Date de publication et mention de la délivrance du brevet: **08.11.2000**
(21) Numéro de dépôt: 95401150.8
(22) Date de dépôt: 17.05.1995
(51) Int. Cl.: A23J 3/34, A23L 1/305, A61K 7/48, A61K 38/01

(54) **Hydrolysat de protéines d'animaux marins, procédé d'obtention et applications**
Proteinhydrolysat aus Seetieren, Verfahren zur Herstellung und Verwendung
Proteinhydrolysate from marine animals, process for obtaining and applications

(30) Priorité: 17.05.1994 FR 9405997
(43) Date de publication de la demande: 22.11.1995
(73) Titulaire: DIELEN LABORATOIRES, 50700 Valognes (FR)
(72) Inventeur: Allaume, Patrick, F-56100 Lorient (FR); Marco, Georges, F-56100 Lorient (FR); Noel, Bernard, F-50100 Cherbourg (FR); Noel, Antoine, F-50100 Cherbourg (FR)
(74) Mandataire: Michelet, Alain

(56) Documents cités:
- EP-A- 0 055 172
- EP-A- 0 106 309
- EP-A- 0 254 289
- WO-A-80/00059
- WO-A-93/10802
- DE-A- 4 119 140
- FR-A- 2 332 027
- FR-A- 2 540 381
- US-A- 4 130 555
- BIOCHIM. BIOPHYS. ACTA, vol. 797, no. 27, 1984 pages 221-230, M. ITO ET AL 'ISOLATION AND CHARACTERIZATION OF AN ASPARAGINE-LINKED KERATAN SULFATE FROM THE SKIN OF A MARINE TELEOST'
- PATENT ABSTRACTS OF JAPAN vol. 011 no. 232 (C-437) ,29 Juillet 1987 & JP-A-62 045678 (TAKIRON CO. LTD.) 27 Février 1987
- DATABASE WPI Week 7744 Derwent Publications Ltd., London, GB; AN 77-78290 & JP-A-52 111 600 (NIPPI KK) , 19 Septembre 1977
- PATENT ABSTRACTS OF JAPAN vol. 014 no. 322 (C-0739) ,10 Juillet 1990 & JP-A-02 115112 (POLA CHEM IND INC) 27 Avril 1990
- DATABASE WPI Week 9502 Derwent Publications Ltd., London, GB; AN 95-009102 & JP-A-06 292 715 (NIPPON HAM KK) , 21 Octobre 1994
- Tome, D. (1990). Le rôle physiologique des peptides issus de la digestion des protéines. La Lettre Scientifique de la Fondation Française pour la Nutrition, Janvier 1990, no. 3

## Description

L'invention concerne un hydrolysat de protéines, provenant de tissus d'animaux marins riches en collagène, ainsi qu'un procédé pour son obtention. Cet hydrolysat trouve application dans des produits et compositions variés, notamment à des fins cosmétiques ou nutritionnelles.

L'hydrolyse de protéines de poissons a été déjà décrite, voir par exemple le brevet FR-2.352.498. Ce document a pour objet un procédé de fabrication de concentré de protéines de poissons par voie enzymatique, en utilisant les enzymes protéolytiques contenus dans les viscères de l'animal. On évite ainsi de recourir à une hydrolyse acide à des températures atteignant 100°C, ces conditions entraînant la destruction des vitamines d'origine ainsi que d'une importante partie des acides aminés des protéines de poissons. L'hydrolysat obtenu selon ce procédé contient des protéines non digérées ainsi que divers acides aminés; les produits correspondants peuvent être utilisés chez l'homme ou l'animal, notamment contre l'asthénie et la perte de poids.

Par ailleurs, un procédé pour la stabilisation des produits nutritionnels liposolubles dégradables a été décrit dans le brevet FR-2.496.408. Ce document propose de mélanger au moins un produit nutritionnel liposoluble dégradable avec , par exemple, un hydrolysat de protéines de poissons. Son but était donc de stabiliser, grâce au protéolysat associé, les produits nutritionnels liposolubles qui ont tendance à s'oxyder et se dégrader très rapidement.

Un procédé de préparation de peptides de faible poids moléculaire après une hydrolyse de diverses protéines est décrit dans la demande de brevet européen n° EP-0 106 309. Ces peptides sont ensuite extraits de l'hydrolysat par une technique séparative, effectuée après une mise en solution hydro-alcoolique, de façon à sélectionner les peptides de faible poids moléculaire, c'est-à-dire ayant un poids moléculaire inférieur à 500 daltons.

Un procédé de préparation d'un hydrolysat de gélatine au goût sucré est décrit dans la demande de brevet français n°76 34 628 (publiée sous le n° 2 332 027). Un tel procédé comprend une étape d'hydrolyse de collagène d'animal, en particulier de la peau de ventre de porc. La préparation finale contient l'hydrolysat de gélatine additionné de tryptophane et d'un édulcorant.

La demande internationale n° WO 80/00059 décrit une hydrolyse chimique en milieu acide de protéines de pied de volailles.

La demande de brevet européen n° EP 0 254 289 concerne des hydrolysats protéiques de peau de boeuf ou de porc comprenant du collagène présentant une masse moléculaire moyenne comprise entre 10 et 80 kD, destinés à la préparation d'agents pour le traitement d'arthroses.

Un procédé pour stimuler la croissance des cellules par action de polypeptides obtenus par hydrolyse de collagène est décrit dans la demande de brevet français n° 83 01 969 (publiée sous le n° 2 540 381).

Selon ce procédé, les polypeptides sont incorporés dans une phase lamellaire lipidique hydratée, telle que des liposomes.

Les collagènes sont des glycoprotéines complexes souvent associées à des glycosamines (chondroitines) dans la formation des édifices de protéoglycannes nécessaires pour le développement ou le maintien des os.

Ces molécules sont également présentes au niveau des tendons, de la peau et des parois aortiques.

Dans l'organisme humain, les glycoprotéines diminuent régulièrement avec l'âge, soit par carence dans la nutrition protéique, soit par déficience dans l'activité des mécanismes enzymatiques qui assurent le renouvellement des collagènes.

En conséquence, on assiste à une diminution du poids du squelette, à des signes de vieillissement au niveau de la peau, ainsi qu'à une fragilisation générale des tissus.

Afin de pallier ces carences, en particulier lorsqu'elles sont osseuses, il est nécessaire d'introduire dans l'organisme du collagène, par l'intermédiaire de précurseurs de plus faible poids moléculaire, ces précurseurs étant capables de transiter aisément à travers la barrière intestinale, et de diffuser ainsi dans l'organisme.

La présente invention a pour but d'apporter sous une forme directement assimilable, soit par l'intestin, soit au niveau des pores de la peau, un hydrolysat comprenant entre autres des précurseurs du collagène.

L'invention a donc pour objet un hydrolysat de tissu ou de cartilage de vertébrés marins riches en collagène, ledit hydrolysat comprenant des peptides, des acides aminés, des protéoglycannes, des oligosaccharides, ainsi que des minéraux, et étant caractérisé en ce que:
- il présente une absence de collagène natif ainsi que de tropocollagène résiduel,
- le profil moléculaire de la fraction protéique présente la répartition suivante:
   . 15 à 40% de molécules de poids moléculaire compris entre 50000 et 10000 daltons
   . 10 à 35% de molécules de poids moléculaire compris entre 10000 et 1800 daltons
   . 10 à 35% de molécules de poids moléculaire compris entre 1800 et 600 daltons
   . 10 à 35% de molécules de poids moléculaire inférieur à 600 daltons.

L'hydrolysat selon l'invention peut posséder également les caractéristiques ci-après, considérées isolément ou selon toutes leurs combinaisons techniquement possibles:
- la teneur en matière azotée totale (N x 6,25) est comprise entre 80 et 95% de l'extrait sec;
- le rapport azote aminé/azote total est compris entre 13 et 20%,
- la teneur en acides aminés libres est comprise entre 20 et 30% de la matière azotée totale,
- la teneur en hydroxyproline et/ou en hydroxylysine est comprise entre 5 et 13% de la matière azotée totale,
- la teneur en glycine est comprise entre 12 et 28% de la matière azotée totale,
- la teneur en protéoglycannes est comprise entre 0,3 et 7% de la matière sèche totale,
- la solubilité dans l'eau est supérieure à 82%, en particulier comprise entre 82 et 90%.
- la teneur en matière lipidique totale est inférieure à 4% de l'extrait sec.

Les molécules protéiques et/ou glycoprotéiques contenues dans les hydrolysats selon l'invention présentent entre autres, les deux spécificités suivantes:
- une répartition en acides aminés identique à celle retrouvée dans le collagène, et ou le cartilage,
- une activité biologique s'exprimant par des propriétés ostéo-inductrices ou régénératrices de l'épiderme.

Préférentiellement les protéoglycannes sont de la famille des glycosamines, tels que la chondroïtine, le kératane et l'acide hyaluronique.

L'invention a pour autre objet un procédé pour l'obtention des hydrolysats de concentrés de protéines d'animaux marins tels que définis ci-dessus, comprenant les étapes successives de:
- sélection des matières premières contenant au moins 25% de collagène et des protéoglycannes, provenant de la peau et/ou du squelette de poissons, de mollusques ou crustacés,
- broyage desdites matières premières,
- filtration,
- addition d'eau, éventuellement acidifiée,
- hydrolyse sous agitation avec apport de protéases
- séparation par filtration ou par centrifugation,
- récupération de l'hydrolysat sous forme de poudre.

Comme indiqué ci-dessus, une caractéristique importante du procédé consiste à sélectionner les matières premières provenant d'animaux marins, pour ne retenir que les matières contenant au moins 25% et de préférence plus de 40% de collagène et de 0,3 à 7% en protéoglycannes. L'homme du métier sait que chaque espèce d'animaux marins possède des collagènes propres à l'espèce, qui sont caractérisés par les taux d'hydroxylysine et/ou d'hydroxyproline dans la matière protéique. La littérature technique et scientifique définit lesdits taux pour les principales espèces d'animaux marins soit pour la peau soit pour le squelette. Ainsi, en fonction de ces données connues, l'homme du métier peut choisir les matières premières ou mélanges de matières premières convenant aux besoins de la présente invention.

L'addition d'eau est avantageusement effectuée à raison de 20 à 25% environ en poids par rapport à la matière première. Si l'on utilise de l'eau acidifiée, l'acide est avantageusement de l'acide chlorhydrique.

Le chauffage met de préférence en oeuvre une température comprise entre 45 et 65°C.

L'hydrolyse sous agitation est réalisée avantageusement à pH constant compris entre 4,5 et 6 pendant une durée comprise entre 2 et 6 heures et à une température de 45°C à 65°C.

L'hydrolyse est conduite par digestion enzymatique contrôlée sous l'action de protéases endogènes, mais également par apport d'enzymes exogènes (protéases). Après l'étape de chauffage, un apport complémentaire de protéases, est ainsi prévu afin de poursuivre l'hydrolyse au-delà de celle réalisée par les protéases endogènes.

Selon la présente description, l'expression "protéases" désigne toute enzyme exogène capable d'hydrolyser les matières premières sélectionnées qui sont soumises au traitement. On donne la préférence aux protéases suivantes: extraits de muqueuses intestinales, extraits pancréatiques, chymosine, trypsine, chymotrypsine, papaïne, seules ou en mélange.

La concentration en protéases dépend de la matière première sélectionnée et de la protéase considérée. Elle est en général de 0,1 à 1% par rapport au poids de la matière première.

L'hydrolyse est poursuivie jusqu'à obtention de l'hydrolysat répondant au(x) profil(s) moléculaire(s) mentionnés précédemment. Un moyen simple d'arrêter l'hydrolyse est de chauffer l'hydrolysat à une température ne dépassant pas 100°C, par exemple de l'ordre de 90°C.

La séparation des différents constituants peut être effectuée par filtration (par exemple sur filtre 400 microns) ou par centrifugation.

La récupération de l'hydrolysat sous forme de poudre peut impliquer des opérations connues de l'homme du métier telles que: concentration sous vide, séchage à basse température, broyage et autres.

En fonction du mode de séparation (filtration ou centrifugation) le conditionnement des produits obtenus se fera respectivement pour une utilisation en nutrition humaine ou animale et une utilisation en cosmétique.

Ce procédé permet d'une part, grâce au choix de matières premières, d'autre part, grâce à l'apport de protéases complémentaires, d'optimiser le taux de peptides précurseurs du collagène dans l'hydrolysat final.

Egalement selon l'invention, des produits à usages nutritionnel et dermatologique, ci-après illustrés par des exemples, peuvent être élaborés à partir des hydrolysats présentement décrits.

L'invention concerne ainsi les applications ci-après:
- un produit nutritionnel comprenant, en plus de l'hydrolysat, un complexe de vitamines A-D3-E, d'oligoéléments et de minéraux,
- un produit nutritionnel conditionné en vue de l'administration par voie orale, par exemple sous forme de gélules contenant la poudre d'hydrolysat seul ou en mélange avec un véhicule pulvérulent, tel que le lactose et/ou avec les compléments vitaminiques, oligoéléments ou minéraux,
- un produit nutritionnel conditionné en vue de l'injection sous-cutanée, en particulier après dilution de la poudre d'hydrolysat dans du sérum physiologique.

En fonction de leur teneur en hydroxylysine ou en hydroxyproline, les produits obtenus ont un intérêt en dermatologie ou en nutrition, pour induire respectivement la reconstitution des os et de la peau chez l'homme.

Ainsi ces produits, selon leurs modes d'application, possèdent les propriétés suivantes:
- Par voie orale:
   Absorption intestinale totale sans avoir recours aux enzymes digestives. Ils comprennent les éléments précurseurs des édifices de protéoglycannes capables d'induire la formation et le développement soit du tissu osseux, soit de la peau.
- Par usage externe:
   Pénétration rapide dans le derme et l'épiderme. Ils vont ainsi contribuer au renouvellement ou à la reconstitution du collagène et/ou du cartilage.

D'autres avantages et caractéristiques de la présente invention ressortiront de la description détaillée ci-après faite en liaison avec divers exemples de fabrication des hydrolysats et des produits correspondants.

### EXEMPLE 1:

Des matières premières obtenues à partir de poissons vivant dans les grandes profondeurs du type "holocephalus bardi" sont triées en fonction de leur teneur en hydroxyproline. On contrôle leur fraîcheur et l'absence de toute contamination bactérienne.

Ces matières premières sont ensuite broyées et mises dans une cuve à hydrolyse classique munie d'une agitation mécanique, puis on ajoute 20% du volume en eau à une température de 60°C. On introduit ensuite de la papaïne à raison de 0,4% en poids et on conduit l'hydrolyse à un pH constant et voisin de 5, pendant une durée de quatre heures, et à une température de 45°C.

Lorsque le rapport azote aminé/azote total atteint 13%, on arrête l'hydrolyse par chauffage à 90°C pendant une durée de 10 minutes.

L'hydrolysat est ensuite centrifugé dans une débourbeuse classique afin d'éliminer les graisses et les particules solides. Le jus obtenu est concentré sous vide, puis séché à basse température par atomisation. Après tamisage et broyage, la poudre obtenue est conditionnée sous emballage étanche.

Des tests ont montré que l'hydrolysat ainsi obtenu est soluble a plus de 88% dans l'eau.

Le produit obtenu a été mélangé à raison de 2% dans un complexe composé des vitamines A-D3-E, d'oligoéléments et de minéraux, qui a été distribué à huit chevaux de course pendant une durée de un mois à raison de 50 g par jour. Les animaux sélectionnés présentaient des troubles de boiterie, avec une légère inflammation au niveau des membres inférieurs (genoux, boulets et sabots) consécutive à une fatigue des tendons et des articulations.

Après dix jours de traitement, les boiteries et inflammations des boulets ont disparu. L'entraînement des animaux a pû être repris après trois semaines d'application, sans avoir recours aux anti-inflammatoires habituels. Après un mois de traitement, les animaux avaient repris la compétition.

### EXEMPLE 2:

On a prélevé à partir de squale, un stock de matières premières dont on a vérifié la teneur en hydroxyproline et hydroxylysine. Après broyage et mélange avec un volume de 25% d'eau à une température de 65°C, on procède à l'hydrolyse telle que décrite dans l'exemple 1, mais en utilisant de la trypsine au lieu de papaïne.

Le jus obtenu est microfiltré sur filtre multicouche à 400 microns, puis concentré sous vide et séché dans une étuve sous vide à une température de dépassant pas 55°C. Le gâteau obtenu est broyé puis conditionné en emballage étanche. L'analyse chimique de l'hydrolysat ainsi obtenu révèle la composition suivante:
87,2% de matière azotée,
4,8% de matière minérale,
3,2% de matière lipidique,
15,8% d'azote aminé/azote total,
5,3% d'hydroxyproline/matière azotée totale,
4,6% d'hydroxylysine/matière azotée totale,
19,8% de glycine/matière azotée totale,
2,3% de protéoglycannes/matière sèche totale
24,2% d'acides aminés libres/matière azotée totale.

Le profil moléculaire de l'hydrolysat présente la répartition suivante:
- 22% de molécules de poids moléculaire compris entre 50000 et 10000 daltons,
- 18% de molécules de poids moléculaire compris entre 10000 et 1800 daltons,
- 30% de molécules de poids moléculaire compris entre 1800 et 600 daltons,
- 30% de molécules de poids moléculaire inférieur à 600 daltons.

Des tests ont montré que cet hydrolysat est soluble à plus de 82,4% dans l'eau.

Le produit a été incorporé à raison de 48% à un mélange de vaseline et de glycérine afin d'obtenir une pommade qui a été appliquée sur des plaies cutanées parallèlement désinfectées, ainsi que sur des brûlures locales. La reconstitution du tissu épidermique a été obtenue en quelques jours.

On a également dilué le produit à raison de 10% dans un sérum physiologique, et on a procédé à des injections sous-cutanées sur des chiens âgés atteints de pelade. Après deux injections à 48 heures d'intervalle, on a obtenu une amélioration très rapide de l'état de la peau.

### EXEMPLE 3

On a prélevé à partir de céphalopodes un stock de squelette dont on a contrôlé la teneur en hydroxylysine par rapport aux protéines totales.

Après broyage et solubilisation sous l'action d'une solution d'acide chlorhydrique 10 N à une température de 65°C, on élève le pH à 5,8 environ. Après décantation, le surnageant déminéralisé est placé dans la cuve d'hydrolyse comme décrit dans les exemples précédents, mais en utilisant un extrait pancréatique à raison de 0,6%.

En fin d'hydrolyse, les protéases sont inactivées par un chauffage à 90°C, puis le jus obtenu est clarifié par ultracentrifugation, et concentré sous vide à 40% ES. Le concentré est séché à l'étuve sous vide et à basse température. Ce concentré peut être également conditionné en ampoule de 10 ml stérilisable pour des injections sous-cutanées.

Après broyage, l'analyse chimique de l'hydrolysat obtenu révèle la composition suivante:
82% de matière azotée,
8,2% de matière minérale,
0,7% de matière lipidique,
18,6% d'azote aminé/azote total,
6,4% d'hydroxyproline/matière azotée totale,
2,8% d'hydroxylysine/matière azotée totale,
26,8% de glycine/matière azotée totale,
5,8% de protéoglycannes/matière sèche totale
22,1% d'acides aminés libres/matière azotée totale.

Le profil moléculaire de la fraction protéique de cet hydrolysat présente la répartition suivante:
- 19% de molécules de poids moléculaire compris entre 50000 et 10000 daltons environ,
- 23% de molécules de poids moléculaire compris entre 10000 et 1800 daltons,
- 32% de molécules de poids moléculaire compris entre 1800 et 600 daltons,
- 26% de molécules de poids moléculaire inférieur à 600 daltons.

Des tests effectués sur cet hydrolysat ont montré qu'il est soluble à plus de 89,1% dans l'eau.

La poudre ainsi obtenue a été mélangée à raison de 30g dans 100g de lactose Codex, puis conditionnée en gélules de 100 mg. Les gélules ont été consommées à raison d'une gélule par jour pendant trois mois par des personnes âgées présentant des signes de décalcification légère, et mises sous régime d'apport contrôlé en calcium à raison de 0,8 mg par jour, et en vitamines D3 (600 UI). A la fin du traitement, une élévation de la masse osseuse effectuée par absorptiomètre biphotonique a permis de vérifier que la perte osseuse était arrêtée; les phosphatases alcalines sériques étaient également en légère augmentation.

L'invention n'est pas limitée aux exemples spécifiquement décrits et diverses modifications peuvent y être apportées sans sortir de son cadre.

## Revendications

1. Hydrolysat de tissu ou de cartilage de vertébrés marins riches en collagène obtenu par digestion enzymatique, ledit hydrolysat comprenant des peptides, des acides aminés, des protéoglycannes ainsi que des minéraux, et étant caractérisé en ce que:
- il présente une absence de collagène et de tropocollagène,
- le profil moléculaire de la fraction protéique présente la répartition suivante:
. 15 à 40% de molécules de poids moléculaire compris entre 50000 et 10000 daltons
. 10 à 35% de molécules de poids moléculaire compris entre 10000 et 1800 daltons
. 10 à 35% de molécules de poids moléculaire compris entre 1800 et 600 daltons.
. 10 à 35% de molécules de poids moléculaire inférieur à 600 daltons.

2. Hydrolysat selon la revendication 1, caractérisé en ce que la teneur en matière azotée totale (N x 6,25) est comprise entre 80 et 95% de l'extrait sec.

3. Hydrolysat selon l'une des revendications 1 ou 2, caractérisé en ce que le rapport azote aminé/azote total est compris entre 13 et 20%.

4. Hydrolysat selon l'une quelconque des revendications 1 à 3, caractérisé en ce que la teneur en acides aminés libres est comprise entre 20 et 30% de la matière azotée totale.

5. Hydrolysat selon l'une quelconque des revendications 1 à 4, caractérisé en ce que la teneur en hydroxyproline et/ou en hydroxylysine est comprise entre 5 et 13% de la matière azotée totale.

6. Hydrolysat selon l'une quelconque des revendications 1 à 5, caractérisé en ce que la teneur en glycine est comprise entre 12 et 28% de la matière azotée totale.

7. Hydrolysat selon l'une quelconque des revendications 1 à 6, caractérisé en ce que la solubilité dans l'eau est supérieure à 82%, en particulier comprise entre 82 et 90%.

8. Hydrolysat selon la revendication 7, caractérisé en ce que la solubilité dans l'eau est supérieure à 88%.

9. Hydrolysat selon l'une quelconque des revendications 1 à 7, caractérisé en ce qu'il comprend:
87,2% de matière azotée,
4,8% de matière minérale,
3,2% de matière lipidique,
15,8% d'azote aminé/azote total,
5,3% d'hydroxyproline/matière azotée totale,
4,6% d'hydroxylysine/matière azotée totale,
19,8% de glycine/matière azotée totale,
2,3% de protéoglycannes/matière sèche totale,
24,2% d'acides aminés libres/matière azotée totale,
et en ce que son profil moléculaire présente la répartition suivante:
- 22% de molécules de poids moléculaire compris entre 50000 et 10000 daltons,
- 18% de molécules de poids moléculaire compris entre 10000 et 1800 daltons environ,
- 30% de molécules de poids moléculaire compris entre 1800 et 600 daltons.
- 30% de molécules de poids moléculaire inférieur à 600 daltons.

10. Hydrolysat selon la revendication 9, caractérisé en ce que la solubilité dans l'eau est supérieure à 82,4%.

11. Hydrolysat selon l'une quelconque des revendications 1 à 7, caractérisé en ce qu'il comprend:
82% de matière azotée,
8,2% de matière minérale,
0,7% de matière lipidique,
18,6% d'azote aminé/azote total,
6,4% d'hydroxyproline/matière azotée totale,
2,8% d'hydroxylysine/matière azotée totale,
26,8% de glycine/matière azotée totale,
5,8% de protéoglycannes/matière sèche totale
22,1% d'acides aminés libres/matière azotée totale,
et en ce que son profil moléculaire de la fraction protéique présente la répartition suivante:
- 19% de molécules de poids moléculaire compris entre 50000 et 10000 daltons,
- 23% de molécules de poids moléculaire compris entre 10000 et 1800 daltons,
- 32% de molécules de poids moléculaire compris entre 1800 et 600 daltons,
- 26% de molécules de poids moléculaire inférieur à 600 daltons,

12. Hydrolysat selon la revendication 11, caractérisé en ce que la solubilité dans l'eau est supérieure à 89,1%.

13. Produit nutritionnel ou dermatologique i comprenant un hydrolysat selon l'une quelconque des revendications 1 à 12.

14. Produit nutritionnel selon la revendication 13 comprenant, en plus de l'hydrolysat, un complexe de vitamines A-D3-E, d'oligoéléments et de minéraux.

15. Produit nutritionnel selon l'une des revendications 13 ou 14, caractérisé en ce qu'il est conditionné en vue de l'administration par voie orale, par exemple sous forme de gélules contenant la poudre d'hydrolysat, seul ou en mélange avec un véhicule pulvérulent, tel que le lactose et/ou avec les compléments vitaminiques, oligoéléments ou minéraux.

16. Procédé pour l'obtention d'hydrolysats selon l'une quelconque des revendications 1 à 12, comprenant les étapes successives de:
- sélection des matières premières contenant au moins 25% de collagène, et de 0,2 à 7% de protéoglycannes, provenant de la peau ou du squelette de poissons, de mollusques ou crustacés,
- broyage desdites matières premières,
- filtration,
- addition d'eau, éventuellement acidifiée,
- hydrolyse sous agitation avec apport de protéases,
- séparation par filtration ou par centrifugation,
- récupération de l'hydrolysat sous forme de poudre.

17. Procédé selon la revendication 16, caractérisé en ce que les matières premières sont sélectionnées de façon à contenir plus de 40% de collagène, et de 0,3 à 7% de protéoglycannes.

## Patentansprüche

1. Hydrolysat von Gewebe oder Knorpel collagenreicher mariner Vertebraten, das durch enzymatische Verdauung erhalten wurde, wobei das Hydrolysat Peptide, Aminosäuren, Proteoglycane sowie Mineralstoffe umfasst und dadurch gekennzeichnet ist, dass:
- es kein Collagen und kein Tropocollagen enthält;
- das Molekulargewichtsprofil der Proteinfraktion die folgende Verteilung aufweist:
- 15 bis 40% Moleküle mit einem Molekulargewicht zwischen 50 000 und 10 000 Dalton;
- 10 bis 35% Moleküle mit einem Molekulargewicht zwischen 10 000 und 1800 Dalton;
- 10 bis 35% Moleküle mit einem Molekulargewicht zwischen 1800 und 600 Dalton;
- 10 bis 35% Moleküle mit einem Molekulargewicht von weniger als 600 Dalton.

2. Hydrolysat gemäß Anspruch 1, dadurch gekennzeichnet, dass der Gesamtgehalt an stickstoffhaltiger Substanz (N x 6,25) zwischen 80 und 95% des Trockenextrakts liegt.

3. Hydrolysat gemäß einem der Ansprüche 1 oder 2, dadurch gekennzeichnet, dass das Verhältnis Aminstickstoff/Gesamtstickstoff zwischen 13 und 20% liegt.

4. Hydrolysat gemäß einem der Ansprüche 1 bis 3, dadurch gekennzeichnet, dass der Gehalt an freien Aminosäuren zwischen 20 und 30% der gesamten stickstoffhaltigen Substanz liegt.

5. Hydrolysat gemäß einem der Ansprüche 1 bis 4, dadurch gekennzeichnet, dass der Gehalt an Hydroxyprolin und/oder Hydroxylysin zwischen 5 und 13% der gesamten stickstoffhaltigen Substanz liegt.

6. Hydrolysat gemäß einem der Ansprüche 1 bis 5, dadurch gekennzeichnet, dass der Gehalt an Glycin zwischen 12 und 28% der gesamten stickstoffhaltigen Substanz liegt.

7. Hydrolysat gemäß einem der Ansprüche 1 bis 6, dadurch gekennzeichnet, dass seine Löslichkeit in Wasser über 82% beträgt und insbesondere zwischen 82 und 90% liegt.

8. Hydrolysat gemäß Anspruch 7, dadurch gekennzeichnet, dass seine Löslichkeit in Wasser über 88% beträgt.

9. Hydrolysat gemäß einem der Ansprüche 1 bis 7, dadurch gekennzeichnet, dass es umfasst:
87,2% stickstoffhaltige Substanz;
4,8% mineralische Substanz;
3,2% lipidische Substanz
15,8% Aminstickstoff/Gesamtstickstoff;
5,3% Hydroxyprolin/gesamte stickstoffhaltige Substanz;
4,6% Hydroxylysin/gesamte stickstoffhaltige Substanz;
19,8% Glycin/gesamte stickstoffhaltige Substanz;
2,3% Proteoglycane/Gesamttrockensubstanz;
24,2% freie Aminosäuren/gesamte stickstoffhaltige Substanz;
und dadurch, dass sein Molekulargewichtsprofil die folgende Verteilung aufweist:
- 22% Moleküle mit einem Molekulargewicht zwischen 50 000 und 10 000 Dalton;
- 18% Moleküle mit einem Molekulargewicht zwischen 10 000 und 1800 Dalton;
- 30% Moleküle mit einem Molekulargewicht zwischen 1800 und 600 Dalton;
- 30% Moleküle mit einem Molekulargewicht von weniger als 600 Dalton.

10. Hydrolysat gemäß Anspruch 9, dadurch gekennzeichnet, dass seine Löslichkeit in Wasser über 82,4% beträgt.

11. Hydrolysat gemäß einem der Ansprüche 1 bis 7, dadurch gekennzeichnet, dass es umfasst:
82% stickstoffhaltige Substanz;
8,2% mineralische Substanz;
0,7% lipidische Substanz
18,6% Aminstickstoff/Gesamtstickstoff;
6,4% Hydroxyprolin/gesamte stickstoffhaltige Substanz;
2,8% Hydroxylysin/gesamte stickstoffhaltige Substanz;
26,8% Glycin/gesamte stickstoffhaltige Substanz;
5,8% Proteoglycane/Gesamttrockensubstanz;
22,1% freie Aminosäuren/gesamte stickstoffhaltige Substanz;
und dadurch, dass das Molekulargewichtsprofil seiner Proteinfraktion die folgende Verteilung aufweist:
- 19% Moleküle mit einem Molekulargewicht zwischen 50 000 und 10 000 Dalton;
- 23% Moleküle mit einem Molekulargewicht zwischen 10 000 und 1800 Dalton;
- 32% Moleküle mit einem Molekulargewicht zwischen 1800 und 600 Dalton;
- 26% Moleküle mit einem Molekulargewicht von weniger als 600 Dalton.

12. Hydrolysat gemäß Anspruch 11, dadurch gekennzeichnet, dass seine Löslichkeit in Wasser über 89,1% beträgt.

13. Nahrungsprodukt oder dermatologisches Produkt, das ein Hydrolysat gemäß einem der Ansprüche 1 bis 12 umfasst.

14. Nahrungsprodukt gemäß Anspruch 13, das außer Hydrolysat einen Komplex der Vitamine A-D3-E, von Spurenelementen und Mineralstoffen umfasst.

15. Nahrungsprodukt gemäß einem der Ansprüche 13 oder 14, dadurch gekennzeichnet, dass es im Hinblick auf die orale Verabreichung konditioniert ist, zum Beispiel in Form von Gelatinekapseln, die das Hydrolysatpulver allein oder im Gemisch mit einem pulverigen Träger, wie Lactose, und/oder mit Zusätzen von Vitaminen, Spurenelementen oder Mineralstoffen enthalten.

16. Verfahren zur Gewinnung von Hydrolysaten gemäß einem der Ansprüche 1 bis 12, das nacheinander die folgenden Schritte umfasst:
- Auswahl von Rohstoffen, die wenigstens 25% Collagen und 0,2 bis 7% Proteoglycane enthalten und die von der Haut oder dem Skelett von Fischen, Mollusken oder Krustentieren stammen;
- Vermahlen der Rohstoffe;
- Filtration;
- Zugabe von gegebenenfalls angesäuertem Wasser;
- Hydrolyse unter Rühren und unter Zugabe von Proteasen;
- Trennung durch Filtration oder durch Zentrifugation;
- Gewinnung des Hydrolysats in Pulverform.

17. Verfahren gemäß Anspruch 16, dadurch gekennzeichnet, dass die Rohstoffe so ausgewählt werden, dass sie mehr als 40% Collagen und 0,3 bis 7% Proteoglycane enthalten.

## Claims

1. Hydrolysate of tissue or cartilage from marine vertebrates with a high content in collagen obtained by enzymatic digestion, said hydrolysate comprising peptides, amino acids, proteoglycans as well as minerals, and being characterized in that:
- it exhibits an absence of collagen and tropocollagen,
- the molecular profile of the protein fraction has the following distribution:
. from 15 to 40% of molecules having a molecular weight of between 50,000 and 10,000 daltons
. from 10 to 35% of molecules having a molecular weight of between 10,000 and 1800 daltons
. from 10 to 35% of molecules having a molecular weight of between 1800 and 600 daltons
. from 10 to 35% of molecules having a molecular weight of less than 600 daltons.

2. Hydrolysate according to claim 1, characterized in that the content of total nitrogen-containing material (N x 6.25) ranges between 80 and 95% of the dry extract.

3. Hydrolysate according to one of claims 1 or 2, characterized in that the amine nitrogen/total nitrogen ratio ranges between 13 and 20%.

4. Hydrolysate according to any one of claims 1 to 3, characterized in that the content of free amino acids ranges between 20 and 30% of the total nitrogen-containing material.

5. Hydrolysate according to any one of claims 1 to 4, characterized in that the hydroxyproline and/or hydroxylysine content ranges between 5 and 13% of the total nitrogen-containing material.

6. Hydrolysate according to any one of claims 1 to 5, characterized in that the glycine content ranges between 12 and 28% of the total nitrogen-containing material.

7. Hydrolysate according to any one of claims 1 to 6, characterized in that the water solubility is greater than 82%, especially ranges between 82 and 90%.

8. Hydrolysate according to claim 7, characterized in that the water solubility is greater than 88%.

9. Hydrolysate according to any one of claims 1 to 7, characterized in that it comprises:
87.2% of nitrogen-containing material,
4.8% of inorganic material,
3.2% of lipid material,
15.8% of amine nitrogen/total nitrogen,
5.3% of hydroxyproline/total nitrogen-containing material,
4.6% of hydroxylysine/total nitrogen-containing material,
19.8% of glycine/total nitrogen-containing material,
2.3% of proteoglycans/total dry material,
24.2% of free amino acids/total nitrogen-containing material,
and in that its molecular profile has the following distribution:
- 22% of molecules having a molecular weight of between 50,000 and 10,000 daltons,
- 18 % of molecules having a molecular weight of between 10,000 and 1800 daltons,
- 30% of molecules having a molecular weight of between 1800 and 600 daltons,
- 30% of molecules having a molecular weight of less than 600 daltons.

10. Hydrolysate according to claim 9, characterized in that the water solubility is greater than 82.4%.

11. Hydrolysate according to any one of claims 1 to 7, characterized in that it comprises:
82% of nitrogen-containing material,
8.2% of inorganic material,
0.7% of lipid material,
18.6% of amine nitrogen/total nitrogen,
6.4% of hydroxyproline/total nitrogen-containing material,
2.8% of hydroxylysine/total nitrogen-containing material,
26.8% of glycine/total nitrogen-containing material,
5.8% of proteoglycans/total dry material,
22.1% of free amino acids/total nitrogen-containing material,
and in that its molecular profile of the protein fraction has the following distribution:
- 19% of molecules having a molecular weight of between 50,000 and 10,000 daltons,
- 23 % of molecules having a molecular weight of between 10,000 and 1800 daltons,
- 32% of molecules having a molecular weight of between 1800 and 600 daltons,
- 26% of molecules having a molecular weight of less than 600 daltons.

12. Hydrolysate according to claim 11, characterized in that the water solubility is greater than 89.1%.

13. Nutritional or dermatological product comprising a hydrolysate according to any one of claims 1 to 12.

14. Nutritional product according to claim 13 comprising, in addition to the hydrolysate, a complex of A-D3-E vitamins, trace elements and minerals.

15. Nutritional product according to one of claims 13 or 14, characterized in that it is packaged for orally administration, for example in the form of capsules containing the hydrolysate powder, alone or in a mixture with a powdered carrier, such as lactose and/or with the vitamin, trace element or mineral supplements.

16. Method for obtaining hydrolysates according to any one of claims 1 to 12, comprising the successive steps of:
- selecting of the raw materials containing at least 25% of collagen, and from 0.2 to 7% of proteoglycans, obtained from the skin or the skeleton of fishes, molluscs or shellfish,
- grinding of said raw materials,
- filtration,
- adding of water, optionally acidified,
- hydrolysis with stirring and protease supply,
- separation by filtration or by centrifugation,
- recovering of the hydrolysate as a powder.

17. Method according to claim 16, characterized in that the raw materials are selected so as to contain more than 40% of collagen, and from 0.3 to 7% of proteoglycans.
